(19) Europäisches Patentamt
European Patent Office
Office européen des brevets

(11) **EP 1 944 608 A1**

(12) **EUROPÄISCHE PATENTANMELDUNG**

(43) Veröffentlichungstag:
**16.07.2008 Patentblatt 2008/29**

(51) Int Cl.:
***G01N 33/22*** *(2006.01)*

(21) Anmeldenummer: **07000426.2**

(22) Anmeldetag: **10.01.2007**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK RS**

(71) Anmelder: **Ermishin, Sergey**
**Moscow 141002 (RU)**

(72) Erfinder: **Ermishin, Sergey**
**Moscow 141002 (RU)**

(74) Vertreter: **Jeck, Anton**
**Patentanwalt,**
**Klingengasse 2/1**
**71665 Vaihingen/Enz (DE)**

(54) **Messverfahren für die physikalisch-chemischen Parameter von Erdgas**

(57) Die Erfindung betrifft ein Verfahren zur Messung von physikalisch-chemischen Parametern des Erdgases. Der Druck und die Temperatur des Erdgases werden gemessen. Der Wert eines Steuerparameters wird bei seiner Verwendung ermittelt. Die physikalisch-chemischen Parameter, die den Parametern des Erdgases entsprechen, werden ausgewählt. Die Daten über die physikalisch-chemischen Parameter des Erdgases werden an eine Informationsausgabeeinrichtung übertragen.

Fig. 5a

Z-faktor entspricht Kompressibilitätsfaktor

EP 1 944 608 A1

Fig. 5b   Z-faktor entspricht Kompressibilitätsfaktor

**Beschreibung**

Die Erfindung bezieht sich auf die Metrologie der Erfassung des Erdgases, Durchflusszähler und Vermessungsknoten, die den Gasdurchsatz, die Menge und den Gasheizwert von Erdgas messen. Die Erfindung kann insbesondere für die Durchführung der Messungen der Standarddichte, des Kohlendioxidgehalts, des Stickstoffgehalts und der Heizkraft des Erdgases unterschiedlicher, physikalischchemischer Zusammensetzung verwendet werden.

Messungen des Durchsatzes und der Menge des Erdgases, die mit Messvorrichtungen und Messsystemen verschiedener Wirkprinzipien durchgeführt werden, hängen von physikalisch-chemischen Parameter des Erdgases (des Komponentenbestands und der Standarddichte) ab, die für die Ermittlung des Kompressibilitätsfaktors des Erdgases notwendig sind. Die Berechnung des Kompressibilitätsfaktors des Erdgases wird vorzugsweise durch das Verfahren "NX19 Mod." berechnet und durch die Zustandsgleichung "GERG-91 Mod.", bestimmt. Für die Berechnung nach diesem Verfahren und dieser Zustandsgleichung können die Messwerte der Dichte bei standardisierten Bedingungen $\rho_c$, des Kohlendioxidgehalts $_{xy}$ und des Stickstoffs $_{xa}$ bei dem unbekannten, vollständigen Komponentenbestand des Erdgases [1-4] angewendet werden.

Außerdem hängt der Preis des Erdgases von seinem Heizkraftwert ab, als dessen Messparameter die höchste und die unterste, spezifische Verbrennungswärme hauptsächlich genommen wird. Zur Bestimmung der höchsten und der untersten spezifischen Verbrennungswärme werden auch physikalisch-chemische Messungen der Zusammensetzung des Erdgases zur Hilfe genommen. Insbesondere sind in [5] Verfahren zur Berechnung der höchsten und der untersten spezifischen Wärme der Verbrennung angegeben, die bei dem unbekannten, vollständigen Komponentenbestand des Erdgases aufgrund von bekannten, physikalisch-chemischen Parametern ($_{xa, xy}$) verwendet werden können, wobei $_{xy, xa}$ die Molbrüche des Kohlendioxids bzw. des Stickstoffs im Erdgas sind.

Bei einem unbekannten Komponentenbestand des Gases kann die höchste $H_{C.B}$ und die unterste $H_{C.H}$ spezifische Verbrennungswärme gemäß den folgenden Formeln (1) bestimmt werden:

$$H_{C.B.} = 92{,}819(0{,}51447\rho_c + 0{,}05603 - 0{,}65689 x_a - x_y)$$

$$H_{C.H.} = 85{,}453(0{,}52190\rho_c + 0{,}04242 - 0{,}65197 x_a - x_y) \qquad (1)$$

Zur Zeit werden die physikalisch-chemischen Messungen der Dichte bei den standardisierten Bedingungen $\rho_c$, des Kohlendioxidgehalts $_{xy}$ und des Stickstoffs $_{xa}$ in der Regel mit Chromatografen (Strom- und Laborchromatografen) durgeführt. Die Chromatografen gehören zu kostenaufwendigen Messvorrichtungen, deren Messgenauigkeit vom Ort des durchgeführten Meßverfahrens abhängig. Außerdem wird für Laborchromatografen die Schaffung von speziellen Laborbedingungen gefordert. Gerade deshalb werden die unmittelbaren Messungen $\rho_c, _{xa}, _{xy}$ in der Regel nur mit genügend großen Gasmessobjekten zur Erfassung des Erdgases mit Hilfe von teuren Stromchromatografen vorgenommen. Ferner tritt eine Abweichung bei der Ermittlung der Parameter $\rho_{c, xa, xy}$ auf Gasmessobjekten auf, bei denen die Messungen der Parameter $\rho_{c, xa, xy}$ durch zeitliche Änderungen der physikalisch-chemischen Eigenschaften des Erdgases verändert werden.

In der Patentschrift RU 2.269.113 ist ein Verfahren zur Ermittlung der Kennwerte der physikalischen Eigenschaften des Erdgases beschrieben, bei dem die Parameter des Erdgases mittels Drosselung geändert werden. Dabei werden der Druck sowie die Temperaturen vor, nach und während der Drosselung gemessen. Auf Grund der gemessenen Werten dieser Parameter werden die Kennwerte der physischen Eigenschaften des Erdgases ermittelt.

Die Nachteile dieses Verfahrens bestehen darin, dass eine Installation von turbulenten und laminaren Drosseln notwendig ist, dass eine Möglichkeit zur Messung der chemischen Parameter des Erdgases (der Gehalte des Kohlendioxids und des Stickstoffs) fehlt und dass zusätzlich große Fehler des Verfahrens durch die Notwendigkeit auftreten, die Gaszufuhr streng einzuhalten.

Der Erfindung liegt die Aufgabe zugrunde, ein Messverfahren vorzuschlagen, mit dem die Bestimmung eines unvollständigen Komponentenbestands des Erdgases (der Dichte bei standardisierten Bedingungen, der Gehalte an Kohlendioxid und Stickstoff) möglich ist, wobei Messwerte des absoluten Drucks und der Temperatur des Erdgases verwendet werden.

Diese Aufgabe wird durch die Merkmale des Anspruches 1 gelöst.

Das technische Ergebnis bei der Verwendung der Erfindung ist die Vereinfachung und die Senkung des Aufwands für die Prozedur der Messungen der Standarddichte, des Gehalts an Kohlendioxid und Stickstoff des Erdgases.

Man erkennt, dass die Erfindung jedenfalls dann verwirklicht ist, wenn folgende Schritte realisiert werden:

- Messung des absoluten Drucks und der Temperatur des Erdgases
- Bestimmung : des Änderungsbereichs der Dichtewerte bei standardisierten Bedingungen des Erdgases; des Änderungsbereichs der Werte des Gehalts an Kohlendioxid im Erdgas; des Änderungsbereichs der Werte des Gehalts an Stickstoffs im Erdgas; des Umfangs der Änderung der Dichtewerte bei Betriebsbedingungen des Erdgases,

- Bildung und Abspeicherung der Dichtewerte bei Betriebsbedingungen des Erdgases mit einem minimalen Schritt im Bereich der Dichteänderung bei Betriebsbedingungen des Erdgases
- im Datenspeicher werden Datenfelder der gegebenen Dichtewerte bei standardisierten Bedingungen des Erdgases mit einem minimalen Schritt im Umfang der Veränderung der Dichtewerte bei standardisierten Bedingungen des Erdgases gebildet
- im Datenspeicher werden Datenfelder der Werte des Gehalts an Kohlendioxid im Erdgas mit einem minimalen Schritt im Bereich der Werteänderung des Gehalts an Kohlendioxid im Erdgas gebildet
- im Datenspeicher werden Datenfelder der Werte des Gehalts an Stickstoff im Erdgas mit einem minimalen Schritt im Umfang der Werteveränderung des Gehalts an Stickstoff im Erdgas gebildet
- für jede Angabe aus den Datenfeldern werden die Dichtewerte bei standardisierten Bedingungen, die Gehalte an Kohlendioxid und Stickstoff des Erdgases und für die gemessenen Werte des absoluten Drucks und der Temperatur das Datenfeld der Werte des Kompressibilitätsfaktors unter Verwendung des Verfahrens zur Berechnung bei einem unbekannten, vollständigen Komponentenbestand des Erdgases ausgerechnet,
- der Wert des Steuerparameters wird für jede Angabe errechnet aus den Datenfeldern der : Werte des Kompressibilitätsfaktors; Dichtewerte des Gases bei Betriebsbedingungen, Dichtewerte des Gases bei standardisierten Bedingungen sowie für die gemessenen Werte des absoluten Drucks und der Temperatur des Gases ,
- der Rechenwert des Steuerparameters und der Wert des Steuerparameters aus den vorgegebenen Angaben werden miteinander verglichen
- es werden ausgewählt: die Angaben aus den Datenfeldern der Dichtewerte bei standardisierten Bedingungen des Erdgases ; der Gehalt an Kohlendioxid und Stickstoff des Erdgases, welcher der minimalen Differenz zwischen dem Rechenwert des Steuerparameters und dem Wert des Steuerparameters aus den vorgegebenen Angaben entspricht
- auf eine Informationsausgabeeinrichtung werden übertragen: die Daten über die gewählten Angaben aus den Datenfeldern der Dichtewerte bei standardisierten Bedingungen des Erdgases ; der Gehalte an Kohlendioxid und Stickstoff des Erdgases, welcher der minimalen Differenz zwischen dem Rechenwert des Steuerparameters und dem Wert des Steuerparameters aus den vorgegebenen Angaben entsprechen
- es wird der Kompressibilitätsfaktor unter Verwendung des Verfahrens zur Berechnung bei einem Unbekannten berechnet, auch der vollständigen Komponentenbestand des Erdgases, falls notwendig, ausgerechnet wird und dass
- das Ergebnis wird er auf die Informationsausgabeeinrichtung übertragen.

Eine weitere zweckmäßige Ausgestaltung der Erfindung sieht vor, dass

- mindestens einmal ein zusätzlicher Steuerparameter erneut ausgewählt wird,
- das Suchverfahren der minimalen Differenz zwischen dem Rechenwert des zusätzlichen Steuerparameters und dem Wert des Steuerparameters, der aus den vorgegebenen Angaben gewonnen wird, wiederholt wird und
- auf die Informationausgabeeinrichtung folgende Daten über die gewählten Angaben aus den Datenfeldern übertragen werden; der Dichtewerte bei standardisierten Bedingungen des Erdgases, der Gehalte an Kohlendioxid und Stickstoff des Erdgases, die der minimalen Differenz zwischen dem Rechenwert des zusätzlichen Steuerparameters und dem Wert des Steuerparameters aus den vorgegebenen Angaben entsprechen.

Gemäß einer weiteren Ausbildung der Erfindung wird/werden

- die Dichte bei standardisierten Bedingungen als Steuerparameter gewählt;
- die Rechendichte bei standardisierten Bedingungen für jede Angabe aus den Datenfeldern der Werte des Kompressibilitätsfaktors, für jede Angabe aus den Datenfeldern der Dichtewerte des Gases bei Betriebsbedingungen, für die gemessenen Werte des absoluten Drucks und der Temperatur des Gases unter Verwendung der Zustandsgleichung des Erdgases

$$\rho_c = \rho \cdot p_c \cdot T \cdot K / (p \cdot T_c)$$

($p_c$ und $T_c$ sind der absolute Druck und die Temperatur der standardisierten Bedingungen) errechnet;
- die Rechendichte bei standardisierten Bedingungen und die Angaben aus den Datenfeldern der Dichtewerte bei standardisierten Bedingungen des Erdgases verglichen,
- die Angaben aus den Datenfeldern der Dichtewerte bei standardisierten Bedingungen des Erdgases, der Gehalte an Kohlendioxid und Stickstoff des Erdgases, die der minimalen Differenz zwischen der Rechendichte bei standardisierten Bedingungen und den Angaben aus den Datenfeldern der Dichtewerte bei standardisierten Bedingungen entsprechen, ausgewählt, und

- die Daten über die gewählten Angaben aus den Datenfeldern der Dichtewerte bei standardisierten und den Betriebsbedingungen des Erdgases auf die Informationsausgabeeinrichtung übertragen, die der minimalen Differenz zwischen der Rechendichte bei standardisierten Bedingungen und den Angaben aus den Datenfeldern der Dichtewerte bei standardisierten Bedingungen entsprechen.

Eine weitere Ausbildung der Erfindung sieht vor, dass

- als zusätzlicher Steuerparameter der Kompressibilitätsfaktor bei standardisierten Bedingungen gewählt wird,
- der Kompressibilitätsfaktor bei standardisierten Bedingungen für die Angaben aus den vorgegebenen Datenfeldern der Dichtewerte des Gases bei standardisierten Bedingungen, der Gasdichte bei Betriebsbedingungen, der Gehalte an Kohlendioxid und Stickstoff des Erdgases unter Verwendung der Formel

$$z_{\mathrm{c}} = 1 - (0{,}0741\rho_{\mathrm{c}} - 0{,}006 - 0{,}063 x_{\mathrm{a}} - 0{,}0575 x_{\mathrm{y}})^2.$$

($x_a$ und $x_y$ sind die Molbrüche des Stickstoffs und des Kohlendioxids im Erdgas) errechnet wird

- der Kompressibilitätsfaktor bei standardisierten Bedingungen gemäß den Rechenwerten der Gasdichte bei standardisierten Bedingungen errechnet wird, und
- die Daten über die gewählten Angaben aus den Datenfeldern der Werte des Gehalts an Kohlendioxid des Erdgases, die der minimalen Differenz für den Kompressibilitätsfaktor nach den vorgegebenen Angaben der Gasdichte bei standardisierten Bedingungen und den Rechenwerten der Gasdichte des Gases bei standardisierten Bedingungen entsprechen, auf die Informationsausgabeeinrichtung übertragen werden.

Eine weitere zweckmäßige Ausbildung der Erfindung sieht vor, dass

- als zusätzlicher Steuerparameter der Kompressibilitätsfaktor des Gases gewählt wird,
- er unter Verwendung der Gleichung des Zustands des Erdgases

$$K = \rho_c \cdot p \cdot T_c / (\rho \cdot p_c \cdot T)$$

($p_c$ und $T_c$ sind der Druck und die Temperatur der standardisierten Bedingungen) für die gemessenen Werte des absoluten Drucks und der Temperatur und der unter Verwendung der Steuerparameter ausgerechneten Dichtewerte bei standardisierten und Betriebsbedingungen des Erdgases ausgerechnet wird,

- das Datenfeld der Werte des Kompressibilitätsfaktors unter Verwendung des Verfahrens zur Berechnung bei einem unbekannten, vollständigen Komponentenbestand des Erdgases für den unter Verwendung der Steuerparameter ausgerechneten Gehalt an Kohlendioxid und der Angaben aus dem Datenfeld der Werte des Gehalts an Stickstoff im Erdgas zusätzlich ausgerechnet wird und
- die Daten über die gewählten Angaben aus dem Datenfeld der Werte des Gehalts an Stickstoff des Erdgases, die der minimalen Differenz des Kompressibilitätsfaktors unter Verwendung der Zustandsgleichung des Erdgases

$$K = \rho_c \cdot p \cdot T_c / (\rho \cdot p_c \cdot T)$$

und des Kompressibilitätsfaktors unter Verwendung des Rechenverfahrens bei einem unbekannten, vollständigen Komponentenbestand des Erdgases entsprechen, auf die Informationsausgabeeinrichtung übertragen werden.

Eine weitere zweckmäßige Ausgestaltung der Erfindung sieht vor, dass

- als Steuerparameter der Kompressibilitätsfaktor des Gases gewählt wird,
- zusätzlich der Rechenfaktor der Kompressibilität des Gases für jede Angabe aus den Datenfeldern der Dichtewerte bei standardisierten Bedingungen, für jede Angabe aus den Datenfeldern der Dichtewerte des Gases bei Betriebsbedingungen, für die gemessenen Werte des absoluten Drucks und der Temperatur des Gases unter Verwendung der Gleichung des Zustands des Erdgases

$$K = \rho_c \cdot p \cdot T_c / (\rho \cdot p_c \cdot T)$$

($p_c$ und $T_c$ sind der Druck und die Temperatur der standardisierten Bedingungen) errechnet wird,

- der zusätzlich ausgerechnete Rechenkoeffizient der Kompressibilität des Gases und die Angaben aus den Datenfeldern der Werte des Kompressibilitätsfaktors, die unter Verwendung des Verfahrens zur Berechnung bei einem unbekannten, vollständigen Komponentenbestand des Erdgases gewonnen werden, verglichen werden,
- die Angaben aus den Datenfeldern der Dichtewerte bei standardisierten Bedingungen des Erdgases, der Gehalte an Kohlendioxid und Stickstoff des Erdgases, die der minimalen Differenz zwischen dem zusätzlich ausgerechneten Rechenkoeffizienten der Kompressibilität des Gases und den Angaben aus den Datenfeldern der Werte des Kompressibilitätsfaktors entsprechen, ausgewählt werden, die unter Verwendung des Verfahrens zur Berechnung bei einem unbekannten, vollständigen Komponentenbestand des Erdgases gewonnen werden, und
- in die Informationsausgabeeinrichtung die Daten über die gewählten Angaben aus den Datenfeldern der Dichtewerte bei standardisierten Bedingungen des Erdgases, der Gehalte an Kohlendioxid und Stickstoff des Erdgases, die der minimalen Differenz zwischen dem zusätzlich ausgerechneten Kompressibilitätsfaktor des Gases und den Angaben aus den Datenfeldern der Werte des Kompressibilitätsfaktors entsprechen, übertragen werden, die unter Verwendung des Verfahrens zur Berechnung bei einem unbekannten, vollständigen Komponentenbestand des Erdgases gewonnen werden.

Bei der Auswahl der Messmittel des Gasdurchsatzes und der Menge des Erdgases in der Praxis wird die Information über den erwarteten Änderungsbereich der physikalisch-chemischen Parameter des Erdgases (der Dichtewerte bei standardisierten Bedingungen des Erdgases, den Änderungsbereich der Werte der Gehalte an Kohlendioxid und Stickstoff) und den erwarteten Bereich der Änderungen des Drucks und der Temperatur im Erfassungsknoten verwendet. Diese Information reicht in der Regel aus, um den erwarteten Bereich der Änderungen der Arbeitsdichte des Erdgases im Erfassungsknoten zu ermitteln.

$$\rho^{\min} = \rho_c^{\min} p^{\min} T_c / (p_c T^{\max} K^{\max}) \quad (1)$$

$$\rho^{\max} = \rho_c^{\max} p^{\max} T_c / (p_c T^{\min} K^{\min}) \quad (2) \, ,$$

wobei $K^{\max}$ und $K^{\min}$ der maximale und der minimale Wert des Kompressibilitätsfaktors des Erdgases im Erfassungsknoten sind.

Außerdem werden bei der Projektierung der Erfassungsknoten des Erdgases verschiedene Arbeitsprinzipien für die spezialisierten Programmkomplexe für die Projektierung (beispielsweise für die Erfassungsknoten aufgrund der standardisierten, verengenden Einrichtungen ; siehe das Programmzertifikat "Gasdurchflußzähler-CT" der Russischen Föderation) angewendet, die im Laufe des Projektierungsprozesses die Arbeitsdichte des Erdgases berechnen.

Ausführungsbeispiele der Erfindung sind in der Zeichnung schematisch dargestellt.

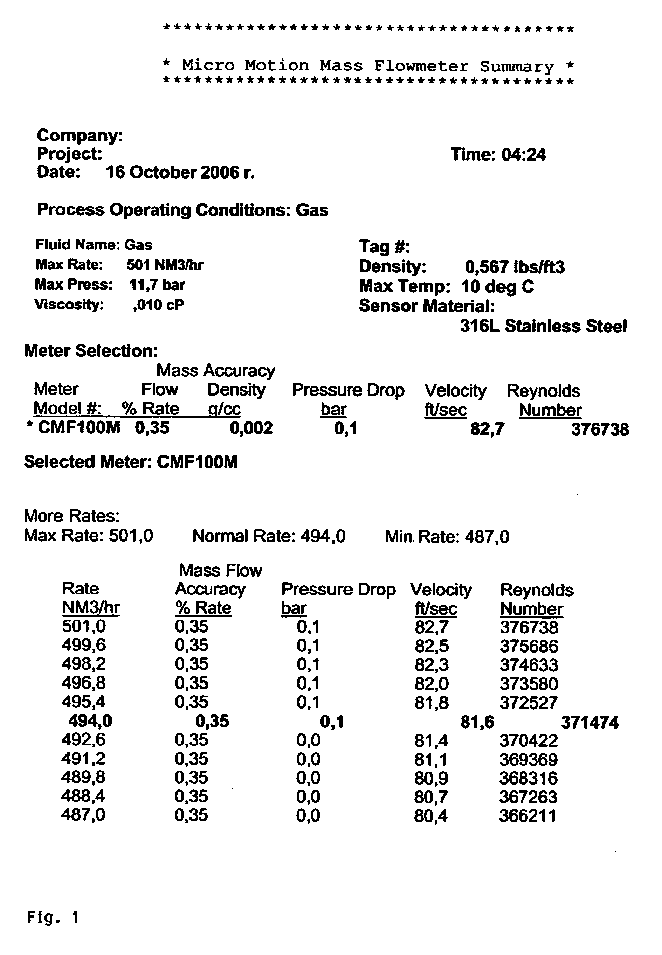

In Fig. 1 ist ein Rechenblatt eines spezialisierbaren Projektierungsprogramms gezeigt, das für die Projektierung der Erfassungsknoten aufgrund der Massendurchflußzähler verwendet wird, wobei aus diesem Rechenblatt die berechneten Kennwerte der Arbeitsdichte des Erdgases im Erfassungsknoten erkennbar sind.

So kann für den Erfassungsknoten des Erdgases der Änderungsbereich der Hauptparameter, die die Zustandsgleichung des Erdgases auswerten, unter Berücksichtigung des Kompressibilitätsfaktors eingegeben werden, der gemäß dem unvollständigen Komponentenbestand errrechnet wird.

Die Hauptidee des Verfahrens gemäß der Erfindung besteht darin, dass die Möglichkeiten der modernen Rechner, die im Erfassungsknoten des Erdgases verwendet werden, ein vollständiges Auslesen (sweeping) der Parameter der Dichte bei den Arbeits- und Standardbedingungen, der Gehalte an Kohlendioxid und Stickstoff bei dem gemessenen Druck und der gemessenen Temperatur erlauben, die die Zustandsgleichung des Erdgases innerhalb der vorgegebenen Änderungsbereiche der auslesbaren Parameter bestimmen, dass beim Erreichen der Konvergenz des Rechenwerts und des Sollwerts des Steuerparameters (beispielsweise der Dichte bei standardisierten Bedingungen, des Kompressibilitätsfaktors bei standardisierten Bedingungen oder des Kompressibilitätsfaktors) die entsprechenden Dichtewerte bei standardisierten Bedingungen, die Gehalte an Kohlendioxid und Stickstoff des Erdgases bestimmt werden können und dass nach dem unvollständigen Komponentenbestand der Kompressibilitätsfaktor des Erdgases errechnet werden kann.

Die Auswahl des Steuerparameters wird durch die Forderung des Vorhandenseins des Extremwerts der Funktion der Differenz des Rechenwerts und Sollwerts des Steuerparameters (die so genannte Aufgabe des statischen Gleichgewichts) begrenzt. In diesem Fall werden die Eigenschaften der Lösung nicht von den Dimensionen der Aufgabe abhängen, und es ist eine Lösung zur Ermittlung der Dichte bei standardisierten Bedingungen, der Gehalte an Kohlendioxid und Stickstoff möglich, die nur dann gewonnen werden kann, wenn die Reihe der Parameter der Aufgabe (insbesondere, die Dichte bei Betriebsbedingungen) nicht gemessen wird. Ein Beispiel solcher Steuerparameter ist die Dichte bei standardisierten Bedingungen.

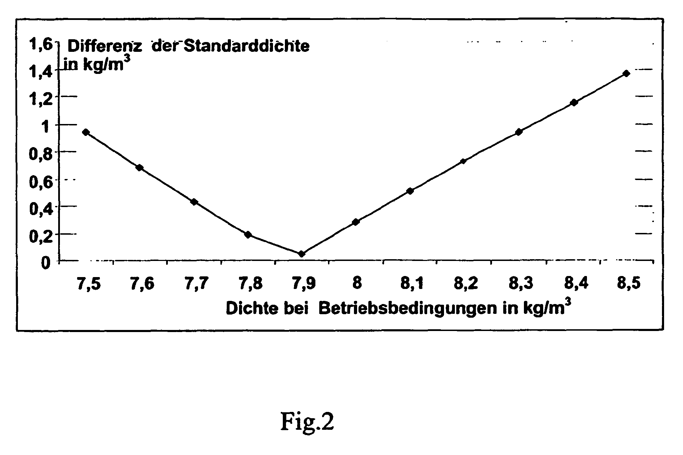

In Fig. 2 ist das Ergebnis der Berechnung des absoluten Werts der Differenz zwischen der Solldichte bei standardisierten Bedingungen für den Schritt des Auslesens der Arbeitsdichte von $0,1 kg/m^3$, die unter Verwendung der Zustandsgleichung des Erdgases berechnet ist, dargestellt:

$$\rho_c = \rho \cdot p_c \cdot T \cdot K / (p \cdot T_c) \qquad (3)$$

wobei $p_c$ und $T_c$ der absolute Druck und die Temperatur der standardisierten Bedingungen sind,

$\rho_c$ - die Solldichte bei standardisierten Bedingungen,
$\rho$ - die Solldichte bei Betriebsbedingungen,
$K$ - der Kompressibilitätsfaktor des Gases, der gemäß dem unvollständigen Komponentenbestand (dem vorgegebenen Gehalt an Kohlendioxid und Stickstoff und der Dichte bei standardisierten Bedingungen) berechnet ist,
$p$ - der gemessene absolute Druck und
$T$ - die gemessene Temperatur des Gases

Wie aus der Grafik in Fig. 2 erkennbar ist, ist hier das Minimum der Differenz des Rechenwerts und des Sollwerts des Steuerparameters (der Dichte bei standardisierten Bedingungen) vorhanden, d.h. die Aufgabe der Suche aus der Zustandsgleichung der Dichte bei standardisierten Bedingungen ist eine Aufgabe des statischen Gleichgewichts, wobei der Charakter der Lage des Minimums nicht von der Aufgabe der Parameter des Auslesens abhängt und den Dichtewert bei standardisierten Bedingungen des Erdgases unabhängig vom Vorhandensein der Information über alle Parameter der Zustandsgleichung des Gases (bei der unbekannten Dichte bei Betriebsbedingungen und des Gehalts an Kohlendioxid und Stickstoff) bestimmt.

Für die Suche der unbekannten Parameter der Zustandsgleichung des Erdgases können Steuerparameter anderer Art angeboten werden, die unterschiedlich zur Dichte bei standardisierten Bedingungen (beispielsweise der Kompressibilitätsfaktor) sind, für die die Forderung des Vorhandenseins des Maximums der Funktion der Differenz des Rechenwerts und des Sollwerts des Steuerparameters erfüllt ist, d.h., bei denen die Aufgabe der Suche der unbekannten Parameter der Zustandsgleichung des Erdgases auf die Aufgabe des statischen Gleichgewichtes zurückbeschränkt ist.

Außerdem kann bei der Suche der unbekannten Parameter der Zustandsgleichung des Erdgases die Verwendung der Steuerparameter verschiedener Art kombiniert werden, d.h. zusätzliche Steuerparameter eingeführt werden, die erlauben, die Suche der anderen, unbekannten Parameter auf die Aufgabe des statistischen Gleichgewichts zu bringen, beispielsweise auf den Gehalt an Kohlendioxid und Stickstoff im Erdgas. Ein solcher zusätzlicher Steuerparameter kann der Kompressibilitätsfaktor bei standardisierten Bedingungen sein:

$$z_c = 1 - (0,0741\rho_c - 0,006 - 0,063x_a - 0,0575x_y)^2. \qquad (4)$$

wobei $x_a$ und $x_y$ die Molbrüche des Stickstoffs und des Kohlendioxids im Erdgas sind.

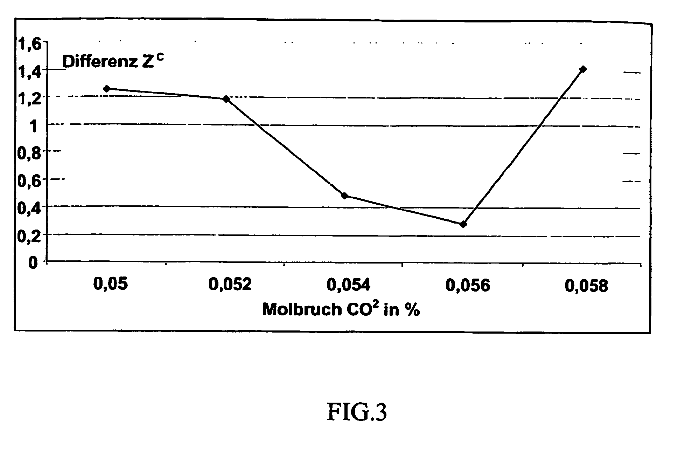

So ist in Fig. 3 das Ergebnis der Berechnung des absoluten Werts der Differenz zwischen dem Kompressibilitätsfaktor bei standardisierten Bedingungen für die Angaben aus den vorgegebenen Datenfeldern der Dichtewerte des Gases bei standardisierten Bedingungen, der Gasdichte bei Betriebsbedingungen, der Gehalte an Kohlendioxid und Stickstoff des Erdgases und dem Kompressibilitätsfaktor bei standardisierten Bedingungen dargestellt, wobei dieses Ergebnis nach den Rechenwerten der Gasdichte bei standardisierten Bedingungen ausgerechnet ist. Das Vorhandensein des Maximums der Differenz des Rechenwerts und des Sollwerts des zusätzlichen Steuerparameters des Kompressibilitätsfaktors bei standardisierten Bedingungen in der Grafik der Fig. 3 ermöglicht, den Gehalt an Kohlendioxid im Erdgas beim Vorhandensein der unbekannten Parameter der Zustandsgleichung des Erdgases zu finden (die Aufgabe der Suche des Gehalts an Kohlendioxid wird auf die Aufgabe des statistischen Gleichgewichts gebracht).

Der offen gebliebene Parameter der Zustandsgleichung des Erdgases - der Gehalt an Stickstoff - wird durch die Ermittlung der minimalen Versetzung zwischen dem Kompressibilitätsfaktor gefunden, der aus der Gleichung des Zustandsgleichung des Erdgases nach folgender Formel ausgerechnet ist:

$$K = \rho_c \cdot p \cdot T_c / (\rho \cdot p_c \cdot T) \qquad (5)$$

und zwar unter Berücksichtigung

- der Messinformation über den absoluten Druck und die Temperatur
- der Suchergebnisse mit Hilfe der Steuerparameter der Gasdichte bei standardisierten und Betriebsbedingungen
- des Verfahrens der Berechnung des Kompressibilitätsfaktors bei unbekanntem, vollständigen Komponentenbestand des Erdgases unter Berücksichtigung der oben genannten Information über die ausgerechneten und gemessenen Parameter sowie
- der Steuerparameter des Gehalts an Kohlendioxid im Gas.

Dieses Berechnungsverfahren zur Berechnung des Kompressibilitätsfaktors bei unbekanntem, vollständigen Komponentenbestand des Erdgases ist beispielsweise die Zustandsgleichung nach GERG-91 Mod. [1 -3]:

$$z = 1 + B_m \rho_M + C_m \rho_M^2 \qquad (6)$$

wobei

$B_m$ und $C_m$    die Koeffizienten GZ sind und
$\rho_M$      die molare Dichte in kmol/m$^3$ ist.
$\rho_M$

Die Koeffizienten der Zustandsgleichung werden nach den folgenden Formeln ermittelt:

$$B_m = x_{\scriptscriptstyle Э}^2 B_1 + x_{\scriptscriptstyle Э} x_a B^*(B_1 + B_2) - 1{,}73 x_{\scriptscriptstyle Э} x_y (B_1 B_3)^{0,5} +$$
$$+ x_a^2 B_2 + 2 x_a x_y B_{23} + x_y^2 B_3, \qquad (7)$$

$$C_m = x_{\scriptscriptstyle Э}^3 C_1 + 3 x_{\scriptscriptstyle Э}^2 x_a C^*(C_1^2 C_2)^{1/3} + 2{,}76 x_{\scriptscriptstyle Э}^2 x_y (C_1^2 C_3)^{1/3} +$$
$$+ 3 x_{\scriptscriptstyle Э} x_a^2 C^*(C_1 C_2^2)^{1/3} + 6{,}6 x_{\scriptscriptstyle Э} x_a x_y (C_1 C_2 C_3)^{1/3} + 2{,}76 x_{\scriptscriptstyle Э} x_y^2 (C_1 C_3^2)^{1/3} +$$
$$+ x_a^3 C_2 + 3 x_a^2 x_y C_{223} + 3 x_a x_y^2 C_{233} + x_y^3 C_3, \qquad (8)$$

wobei $x_{\scriptscriptstyle Э}$ der Molbruch des äquivalenten Kohlenwasserstoffs ist,

$$x_{\scriptscriptstyle Э} = 1 - x_a - x_y \qquad (9)$$

$$B_1 = -0,425468 + 2,865 \cdot 10^{-3} T - 4,62073 \cdot 10^{-6} T^2 +$$
$$+ (8,77118 \cdot 10^{-4} - 5,56281 \cdot 10^{-6} T + 8,8151 \cdot 10^{-9} T^2) H +$$
$$+ (-8,24747 \cdot 10^{-7} + 4,31436 \cdot 10^{-9} T - 6,08319 \cdot 10^{-12} T^2) \times H^2,$$

$$B_2 = -0,1446 + 7,4091 \cdot 10^{-4} T - 9,1195 \cdot 10^{-7} T^2, \qquad (11)$$

$$B_{23} = -0,339693 + 1,61176 \cdot 10^{-3} T - 2,04429 \cdot 10^{-6} T^2, \qquad (12)$$

$$B_3 = -0,86834 + 4,0376 \cdot 10^{-3} T - 5,1657 \cdot 10^{-6} T^2, \qquad (13)$$

$$C_1 = -0,302488 + 1,95861 \cdot 10^{-3} T - 3,16302 \cdot 10^{-6} T^2 +$$
$$+ (6,46422 \cdot 10^{-4} - 4,22876 \cdot 10^{-6} T + 6,88157 \cdot 10^{-9} T^2) H +$$
$$+ (-3,32805 \cdot 10^{-7} + 2,2316 \cdot 10^{-9} T - 3,67713 \cdot 10^{-12} T^2) \times H^2, \qquad (14)$$

$$C_2 = 7,8498 \cdot 10^{-3} - 3,9895 \cdot 10^{-5} T + 6,1187 \cdot 10^{-8} T^2, \qquad (15)$$

$$C_3 = 2,0513 \cdot 10^{-3} + 3,4888 \cdot 10^{-5} T - 8,3703 \cdot 10^{-8} T^2, \qquad (16)$$

$$C_{223} = 5,52066 \cdot 10^{-3} - 1,68609 \cdot 10^{-5} T + 1,57169 \cdot 10^{-8} T^2, \qquad (17)$$

$$C_{233} = 3,58783 \cdot 10^{-3} + 8,06674 \cdot 10^{-6} T - 3,25798 \cdot 10^{-8} T^2, \qquad (18)$$

$$B^* = 0,72 + 1,875 \cdot 10^{-5} (320 - T)^2, \qquad (19)$$

$$C^* = 0,92 + 0,0013 (T - 270). \qquad (20)$$

In den Formelen (10), (14) wird *H* nach folgendem Ausdruck berechnet:

$$H = 128,64 + 47,479 M_э, \qquad (21)$$

wobei $M_3$ die molare Masse des äquivalenten Kohlenwasserstoffs ist, deren Wert aus dem folgenden Ausdruck ermittelt wird:

$$M_3 = (24{,}05525 z_c \rho_c - 28{,}0135 x_a - 44{,}01 x_y)/x_3. \quad (22)$$

Im Ausdruck (22) wird der Molbruch des äquivalenten Kohlenwasserstoffs ($x_3$) unter Verwendung der Formel (9) berechnet, und der Kompressibilitätsfaktor bei standardisierten Bedingungen ($z_c$) wird nach der Formel (4) berechnet.

Nach der Ermittlung der Koeffizienten der Zustandsgleichung (6) $B_m$ wird der Kompressibilitätsfaktor bei dem vorgegeben Druck ($p$, MΠa) und Temperatur ($T$) nach der folgenden Formel $C_m$ berechnet:

$$z = (1 + A_2 + A_1 / A_2)/3, \quad (23)$$

wobei

$$A_2 = \left[ A_0 - (A_0^2 - A_1^3)^{0,5} \right]^{1/3}, \quad (24)$$

$$A_0 = 1 + 1{,}5(B_0 + C_0), \quad (25)$$

$$A_1 = 1 + B_0, \quad (26)$$

$$B_0 = b B_m, \quad (27)$$

$$C_0 = b^2 C_m, \quad (28)$$

$$b = 10^3 p/(2{,}7715 T). \quad (29)$$

ist.

Der Kompressibilitätsfaktor des Erdgases wird nach folgender Formel (30) berechnet:

$$K = z/z_c \quad (30)$$

Das erfindungsgemäße Verfahren gemäß wird nun am folgenden Beispiel näher betrachtet, wobei die Dichte bei standardisierten Bedingungen $\rho_c$ als Steuerparameter gewählt wird.

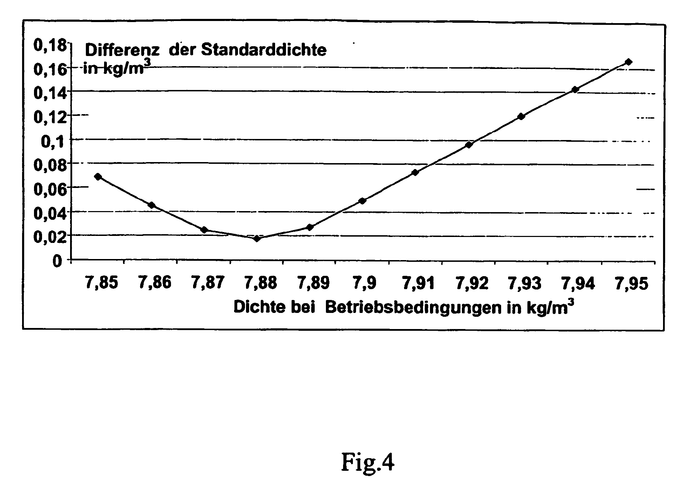

Für die Senkung der Rechenkosten kann das Auslesen (sweeping) innerhalb der vorgegebenen Änderungsbereiche der Parameter der Dichte $p^i$ nicht mit dem minimalen Schritt, aber etappenweise verwirklicht werden: zunächst nach den Einheiten (es wird die beste Lösung mit einer Genauigkeit von 1kg/m$^3$ gesucht), später nach den Zehntelanteilen (es wird die beste Lösung mit einer Genauigkeit von 0,1kg/m$^3$ gesucht), weiter nach den Hundertsteln usw. Der Charakter der Lage des Minimums hängt vom Schritt des Auslesens nicht ab, der in diesem Fall nur die Genauigkeit des erhaltenen Auffindens des Parameters (der Dichte bei standardisierten Bedingungen) bestimmt. So zeigt die Grafik in Fig. 4 die Lage des Minimums der Differenz zwischen der vorgegebenen und gerechneten Dichte bei standardisierten Bedingungen für den Schritt des Auslesens einer Arbeitsdichte von 0,01kg/m³. Der Vergleich der Grafiken der Fig. 2 und der Fig. 4 bestätigt die Tatsache der Unabhängigkeit der Lage des Minimums vom Schritt des Auslesens, der mit den Eigenschaften der Aufgabe des statistischen Gleichgewichts bestimmt wird.

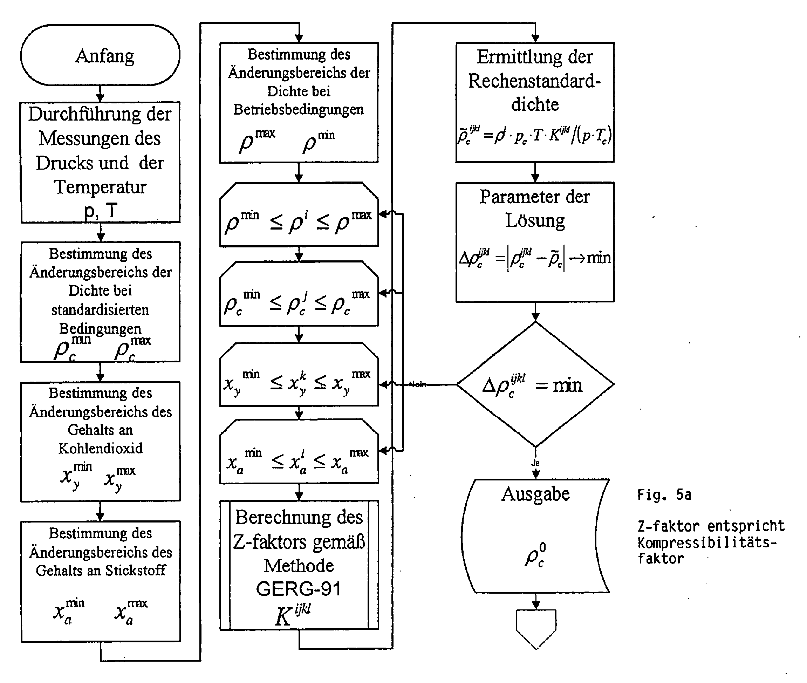

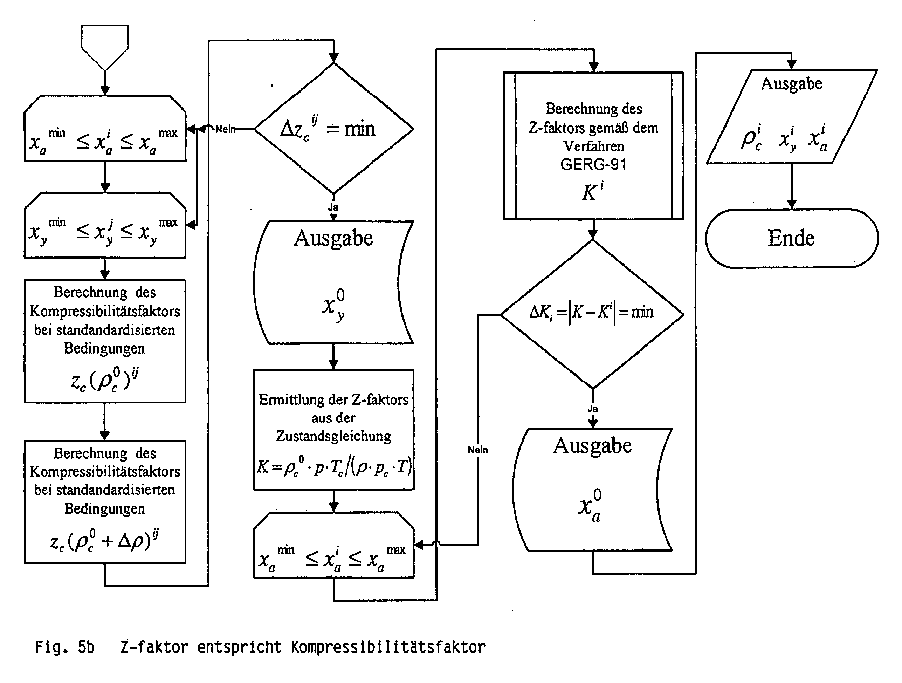

Die Blockschaltung des Beispiels der Realisierung des Verfahrens gemäß der Erfindung ist in Fig. 5 dargestellt.

In der ersten Etappe der Realisierung des Verfahrens werden die Werte des Drucks p und der Temperatur T im Erfassungsknoten des Erdgases gemessen. Weiter werden aufgrund der Projektinformation in den Erfassungsknoten die Änderungsbereiche der Dichte bei standardisierten Bedingungen $\rho_c^{min}$ $\rho_c^{max}$, der Gehalte an Kohlendioxides $x_y^{min}$, $x_y^{max}$ und Stickstoff $x_a^{min}$, $x_a^{max}$ im Erdgas und der Dichte $\rho^{min}$, $\rho^{max}$ bei Betriebsbedingungen festgelegt. Weiter werden die im Erfassungsknoten des Erdgases festgehaltenen, gemessenen Werte des Drucks p und die Temperatur T innerhalb der vorgegebenen Änderungsbereiche der Parameter der Dichte $\rho^i$ bei Betriebsbedingungen, der Dichte bei standardisierten Bedingungen $\rho_c^i$, der Gehalte an Kohlendioxid $x_y^i$ und Stickstoff $x_a^i$ vollständig ausgelesen.

Für die gemessenen Werte des Drucks p und der Temperatur T und jedes aus den ausgelesenen Werten der Parameter der Dichte $\rho^i$ bei den Betriebsbedingungen, der Dichte bei den standardisierten Bedingungen $\rho_c^j$, der Gehalte an Kohlendioxid $x_y^k$ und Stickstoff $x_a^l$ wird der Kompressibilitätsfaktor unter Verwendung des Berechnungsverfahrens des Kompressibilitätsfaktors $K^{ijkl}$ bei unbekanntem, vollständigen Komponentenbestand des Erdgases, beispielsweise die Zustandsgleichung nach GERG-91 (Formeln6, 4, 30) ausgerechnet. In der nächsten Etappe wird bei den gemessenen Werte des Drucks p und der Temperatur T für jeden Wert des Kompressibilitätsfaktors $K^{ijkl}$ und der ausgelesenen Werte der Parameter die Rechendichte bei standardisierten Bedingungen $\widetilde{\rho}_c^{ijkl}$ unter Verwendung der Zustandsgleichung des Erdgases (Formel 3) berechnet. Danach wird das Minimum des Parameters gesucht (Figuren 2 und 4):

$$\Delta\rho_c^{ijkl} = \left| \rho_c^{ijkl} - \widetilde{\rho}_c \right| \to \min \qquad (31)$$

Die im laufenden Zyklus vorgegebene Standarddichte, die mit dem Minimum der Formel (31) $\rho_c^0$ ermittelt wird, ist die gesuchte Dichte bei standardisierten Bedingungen des Erdgases, die aus der Zustandsgleichung auf die Aufgabe des statistischen Gleichgewichts gebracht wird.

In der nächsten Etappe wird die Ermittlung des Gehaltes an Kohlendioixd im ErdGas vorgenommen. Dazu wird das Auslesen der Werte der Parameter der Gehalte an Stickstoff $x_a^i$ und Kohlenidoxid $x_y^j$ und die Berechnung des Kompressibilitätsfaktors (4) bei standardisierten Bedingungen $z_c$ für die gefundene Standarddichte des Erdgases $\rho_c^0$:

$$z_c(\rho_c^0)^{ij} = 1 - (0{,}0741\rho_c^0 - 0{,}006 - 0{,}063x_a^i - 0{,}0575x_y^j)^2 \qquad (32)$$

und für die standardisierte Rechendichte des Erdgases unter Berücksichtigung der ihr entsprechenden Versetzung vorgenommen:

$$z_c(\rho_c^0 + \Delta\rho)^{ij} = 1 - (0{,}0741(\rho_c^0 + \Delta\rho) - 0{,}006 - 0{,}063x_a^i - 0{,}0575x_y^j)^2 \qquad (33)$$

wobei $x_a$ und $x_y$ die Molbrüche des Stickstoffs und des Kohlendioxides im Erdgas sind.

In der nächsten Etappe des Algorithmus wird die Suche des minimalen Werts der Differenz ( Fig. 3) vorgenommen:

$$\Delta z^{ij} = \left| z_c (\rho_c^0)^{ij} - z_c (\rho_c^0 + \Delta\rho)^{ij} \right| \rightarrow \min \qquad (34)$$

Der dem Minimum des Ausdrucks (34) entsprechende Wert des Molbruchs des Kohlendioxids $x_y^0$ ist eine gesuchte Größe des Gehalts an Kohlendioxid im Erdgas, die aus der Berechnungsgleichung für den Kompressibilitätsfaktor ermittelt wird, der auf die Aufgabe des statistischen Gleichgewichts gebracht ist.

In der nächsten Etappe wird die Ermittlung des Gehalts an Stickstoff im Erdgas vorgenommen. Dazu wird am Anfang der Kompressibilitätsfaktor nach der Formel (5) für die gefundene Standarddichte des Erdgases $\rho_c^0$ t ausgerechnet:

$$K = \rho_c^{\ 0} \cdot p \cdot T_c \big/ \left( \rho \cdot p_c \cdot T \right) \qquad (35)$$

Das Auslesen (sweeping) der Werte der Parameter des Gehalts an Stickstoff $x_a^i$ und die Berechnung des Kompressibilitätsfaktors $K^i$ bei dem unbekannten, vollständigen Komponentenbestand des Erdgases werden vorgenommen, wobei beispielsweise die Zustandsgleichung nach GERG-91 (Formeln 6, 4, 30) bei dem früher erhaltenen Wert des Molbruchs des Kohlendioxids $x_y^0$ , und der Standarddichte des Erdgases $\rho_c^0$ verwendet wird. Die Suche des minimalen Werts der Differenz

$$\Delta K_i = \left| K - K^i \right| = \min \qquad (36)$$

erlaubt, den Wert des Molbruchs des Stickstoffs $x_a^0$ zu ermitteln, der eine gesuchte Größe des Gehalts an Stickstoff im Erdgas ist.

Die im Laufe des Berechnungsprozesses ermittelten Werte der Dichte bei standardisierten Bedingungen $\rho_c^0$ , die Molbrüche des Kohlendioxids $x_y^0$ und des Stickstoffs $x_a^0$ werden auf die Informationsausgabeeinrichtung übertragen.

Aufgrund der gegebenen, physikalisch-chemischen Parameter wird notwendigerweise die Berechnung des Kompressibilitätsfaktors nach der Formel (35) und der Dichte bei Betriebsbedingungen Vorgenommen:

$$\rho = \rho_c \cdot p \cdot T_c \big/ \left( p_c \cdot T \cdot K \right) \qquad (37)$$

Literaturverzeichnis

1. GOST 30319.2-9" Erdgas, "Verfahren zur Berechnung der physischen Eigenschaften, Ermittlung des Kompressibilitätsfaktors"

2. Jaeschke M., Humphreys A.E., "Standard GERG Virial Equation for Field Use, Simplification of the Input Data Requirements for the GERG Virial Equation, an Alternative Means of Compressibility Factor Calculation for Natural Gases and Similar Mixtures", GERG TM5, 1991, GERG Technical Monograph, 1991, 173 Seiten

3. ICO/TC 193 SC1163, "Natural gas - calculation of compression factor, Part 3: Calculation using measured physical properties".

4. VDI/VDE 2040, part 2, 1987, "Calculation principles for measurement of fluid flow using orifice plates, nozzles and venturi tubes,. Equations and formulas".

5. GOST 30319.1-96, "Erdgas, Die Methoden der Berechnung der physischen Eigenschaften, die Bestimmung der physischen Eigenschaften des Erdgases, seiner Komponenten und der Produkte seiner Verarbeitung"

**Patentansprüche**

1. Verfahren zur Messung von physikalisch-chemischen Parametern des Erdgases,
   **dadurch gekennzeichnet,**
   **dass** der Druck und die Temperatur des Erdgases gemessen werden, dass der Wert eines Steuerparameters bei seiner Verwendung ermittelt wird, dass die physikalisch-chemischen Parameter, die den Parametern des Erdgases entsprechen, ausgewählt werden und dass die Daten über die physikalisch-chemischen Parameter des Erdgases an eine Informationsausgabeeinrichtung übertragen werden.

2. Verfahren nach Anspruch 1,
   **dadurch gekennzeichnet,**
   **dass** mindestens einmal ein zusätzlicher Steuerparameter erneut gewählt wird und dass die Prozedur der Wahl der physikalisch-chemischen Parameter entsprechend den Parametern des Erdgases wiederholt wird.

3. Verfahren nach Anspruch 1 oder 2,
   **dadurch gekennzeichnet,**
   **dass** als Steuerparameter die Dichte bei standardisierten Bedingungen gewählt wird.

4. Verfahren nach Anspruch 3,
   **dadurch gekennzeichnet,**
   **dass** als Wert des zusätzlichen Steuerparameters der Kompressibilitätsfaktor bei standardisierten Bedingungen gewählt wird.

5. Verfahren nach Anspruch 4,
   **dadurch gekennzeichnet,**
   **dass** als zusätzlicher Steuerparameter der Kompressibilitätsfaktor des Erdgases gewählt wird.

6. Verfahren nach Anspruch 1 oder 2,
   **dadurch gekennzeichnet,**
   **dass** als Steuerparameter der Kompressibilitätsfaktor des Erdgases gewählt wird.

```
***************************************

* Micro Motion Mass Flowmeter Summary *
***************************************
```

**Company:**
**Project:**                                    **Time: 04:24**
**Date:    16 October 2006 r.**

**Process Operating Conditions: Gas**

Fluid Name: Gas                          **Tag #:**
Max Rate:    501 NM3/hr                   **Density:    0,567 lbs/ft3**
Max Press:   11,7 bar                     **Max Temp: 10 deg C**
Viscosity:   ,010 cP                      **Sensor Material:**
                                         **316L Stainless Steel**

**Meter Selection:**

| Meter Model #: | Mass Flow % Rate | Accuracy Density g/cc | Pressure Drop bar | Velocity ft/sec | Reynolds Number |
|---|---|---|---|---|---|
| * CMF100M | 0,35 | 0,002 | 0,1 | 82,7 | 376738 |

**Selected Meter: CMF100M**

**More Rates:**
Max Rate: 501,0        Normal Rate: 494,0        Min. Rate: 487,0

| Rate NM3/hr | Mass Flow Accuracy % Rate | Pressure Drop bar | Velocity ft/sec | Reynolds Number |
|---|---|---|---|---|
| 501,0 | 0,35 | 0,1 | 82,7 | 376738 |
| 499,6 | 0,35 | 0,1 | 82,5 | 375686 |
| 498,2 | 0,35 | 0,1 | 82,3 | 374633 |
| 496,8 | 0,35 | 0,1 | 82,0 | 373580 |
| 495,4 | 0,35 | 0,1 | 81,8 | 372527 |
| **494,0** | **0,35** | **0,1** | **81,6** | **371474** |
| 492,6 | 0,35 | 0,0 | 81,4 | 370422 |
| 491,2 | 0,35 | 0,0 | 81,1 | 369369 |
| 489,8 | 0,35 | 0,0 | 80,9 | 368316 |
| 488,4 | 0,35 | 0,0 | 80,7 | 367263 |
| 487,0 | 0,35 | 0,0 | 80,4 | 366211 |

Fig. 1

Fig.2

FIG.3

Fig.4

Fig. 5a

Z-faktor entspricht
Kompressibilitätsfaktor

EP 1 944 608 A1

$$x_a^{min} \leq x_a^i \leq x_a^{max}$$

$$x_y^{min} \leq x_y^j \leq x_y^{max}$$

Berechnung des
Kompressibilitätsfaktors
bei standandardisierten
Bedingungen

$$z_c(\rho_c^0)^{ij}$$

Berechnung des
Kompressibilitätsfaktors
bei standandardisierten
Bedingungen

$$z_c(\rho_c^0 + \Delta\rho)^{ij}$$

$$\Delta z_c^{ij} = min$$

Nein / Ja

Ausgabe

$$x_y^0$$

Ermittlung der Z-faktors
aus der
Zustandsgleichung

$$K = \rho_c^0 \cdot p \cdot T_c / (\rho \cdot p_c \cdot T)$$

$$x_a^{min} \leq x_a^i \leq x_a^{max}$$

Berechnung des
Z-faktors gemäß dem
Verfahren
GERG-91

$$K^i$$

$$\Delta K_i = |K - K^i| = min$$

Nein / Ja

Ausgabe

$$x_a^0$$

Ausgabe

$$\rho_c^i \quad x_y^i \quad x_a^i$$

Ende

Fig. 5b    Z-faktor entspricht Kompressibilitätsfaktor

19

**Europäisches Patentamt**

## EUROPÄISCHER RECHERCHENBERICHT

Nummer der Anmeldung

EP 07 00 0426

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 4 584 868 A (JACOBSEN ROBERT S [US] ET AL) 29. April 1986 (1986-04-29) * Anspruch 1 * | 1-6 | INV. G01N33/22 |
| X | DATABASE WPI Week 198030 Derwent Publications Ltd., London, GB; AN 1980-52973C XP002443961 -& SU 702 267 A (VNIIGAZ NATURAL GAS) 7. Dezember 1979 (1979-12-07) * das ganze Dokument * | 1-6 | |
| X | EP 0 608 736 A2 (BADGER METER INC [US] INSTROMET INC [US]) 3. August 1994 (1994-08-03) * das ganze Dokument * | 1-6 | |
| A | US 5 237 852 A (KOLPAK MIROSLAV M [US]) 24. August 1993 (1993-08-24) * das ganze Dokument * | 1-6 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| München | 24. Juli 2007 | Thomte, Peter |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

&  : Mitglied der gleichen Patentfamilie, übereinstimmendes
     Dokument

EPO FORM 1503 03.82 (P04C03)

**EP 1 944 608 A1**

**ANHANG ZUM EUROPÄISCHEN RECHERCHENBERICHT
ÜBER DIE EUROPÄISCHE PATENTANMELDUNG NR.**

EP 07 00 0426

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-07-2007

| Im Recherchenbericht angeführtes Patentdokument | | Datum der Veröffentlichung | Mitglied(er) der Patentfamilie | | Datum der Veröffentlichung |
|---|---|---|---|---|---|
| US 4584868 | A | 29-04-1986 | AU | 573945 B2 | 23-06-1988 |
| | | | AU | 5242986 A | 20-11-1986 |
| | | | BR | 8602418 A | 21-01-1987 |
| | | | CN | 86101086 A | 12-11-1986 |
| | | | DE | 208046 T1 | 11-06-1987 |
| | | | DK | 54386 A | 17-11-1986 |
| | | | EP | 0208046 A1 | 14-01-1987 |
| | | | JP | 61265548 A | 25-11-1986 |
| SU 702267 | A | 05-12-1979 | KEINE | | |
| EP 0608736 | A2 | 03-08-1994 | DE | 69425515 D1 | 21-09-2000 |
| | | | DK | 608736 T3 | 09-10-2000 |
| | | | ES | 2149219 T3 | 01-11-2000 |
| US 5237852 | A | 24-08-1993 | KEINE | | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- RU 2269113

### In der Beschreibung aufgeführte Nicht-Patentliteratur

- Verfahren zur Berechnung der physischen Eigenschaften, Ermittlung des Kompressibilitätsfaktors. *GOST 30319.2-9*
- **JAESCHKE M. ; HUMPHREYS A.E.** Standard GERG Virial Equation for Field Use, Simplification of the Input Data Requirements for the GERG Virial Equation, an Alternative Means of Compressibility Factor Calculation for Natural Gases and Similar Mixtures. *GERG TM5, 1991, GERG Technical Monograph,* 1991, 173

- Natural gas - calculation of compression factor, Part 3: Calculation using measured physical properties. *ICO/TC 193 SC1163*
- Calculation principles for measurement of fluid flow using orifice plates, nozzles and venturi tubes,. Equations and formulas. *VDI/VDE 2040,* 1987
- Erdgas, Die Methoden der Berechnung der physischen Eigenschaften, die Bestimmung der physischen Eigenschaften des Erdgases, seiner Komponenten und der Produkte seiner Verarbeitung. *GOST 30319.1-96*